(19) Europäisches Patentamt
European Patent Office
Office européen des brevets

(11) EP 0 840 794 B1

(12) EUROPÄISCHE PATENTSCHRIFT

(45) Veröffentlichungstag und Bekanntmachung des Hinweises auf die Patenterteilung:
**15.02.2006 Patentblatt 2006/07**

(21) Anmeldenummer: **96909160.2**

(22) Anmeldetag: **01.04.1996**

(51) Int Cl.:
***C12N 15/85*** (2006.01) ***C07K 14/035*** (2006.01)
***A61K 39/245*** (2006.01) ***A61K 48/00*** (2006.01)

(86) Internationale Anmeldenummer:
**PCT/EP1996/001428**

(87) Internationale Veröffentlichungsnummer:
**WO 1997/005262 (13.02.1997 Gazette 1997/08)**

(54) **ARZNEIMITTEL ENTHALTEND WENIGSTENS EINEN TEIL DES UL84-PROTEINS DES CYTOMEGALOVIRUS, VERWENDUNG VON POLYPEPTIDEN ENTSPRECHEND DER AMINOSÄURESEQUENZ DES UL84-PROTEINS UND VERFAHREN ZUR EINFÜHRUNG VON UL84 IN ZIELZELLEN**

MEDICAMENT CONTAINING AT LEAST ONE PART OF THE UL84 PROTEIN OF THE CYTOMEGALOVIRUS, USE OF POLYPEPTIDES CORRESPONDING TO THE AMINO ACID SEQUENCE OF THE UL84 PROTEIN, AND PROCESS FOR INTRODUCING UL84 INTO TARGET CELLS

MEDICAMENT CONTENANT AU MOINS UNE PARTIE DE LA PROTEINE UL84 DU CYTOMEGALOVIRUS, UTILISATION DE POLYPEPTIDES CONFORMEMENT A LA SEQUENCE D'AMINOACIDES DE LA PROTEINE UL 84, ET PROCEDE D'INTRODUCTION DE UL84 DANS DES CELLULES CIBLES

(84) Benannte Vertragsstaaten:
**AT BE CH DE DK ES FI FR GB GR IE IT LI LU MC NL PT SE**

(30) Priorität: **25.07.1995 DE 19527129**

(43) Veröffentlichungstag der Anmeldung:
**13.05.1998 Patentblatt 1998/20**

(73) Patentinhaber: **Chiron Behring GmbH & Co.
35006 Marburg (DE)**

(72) Erfinder:
- **STAMMINGER, Thomas
D-90522 Oberasbach (DE)**
- **PLACHTER, Bodo
D-55101 Mainz (DE)**

(74) Vertreter: **Hallybone, Huw George et al
Carpmaels and Ransford,
43 Bloomsbury Square
London WC1A 2RA (GB)**

(56) Entgegenhaltungen:
**EP-A- 0 534 102**

- **INTERVIROLOGY (1993), VOLUME DATE 1992, 34 (2), 94-104, 1993, XP000578142 YUO, CHUNG YEE ET AL: "Stable expression of functional human cytomegalovirus immediate-early proteins IE1 and IE2 in HeLa cells"**
- **J VIROL 66 (2). 1992. 1098-1108, XP000578057 HE Y S ET AL: "CHARACTERIZATION OF HUMAN CYTOMEGALOVIRUS UL84 EARLY GENE AND IDENTIFICATION OF ITS PUTATIVE PROTEIN PRODUCT."**
- **JOURNAL OF VIROLOGY 68 (11). 1994. 7549-7553, XP002010431 SPECTOR D J ET AL: "Protein-protein interactions between human cytomegalovirus IE2-580aa and pUL84 in lytically infected cells."**
- **SCIENCE, Bd. 265, Nr. 5177, 2.September 1994, LANCASTER, PA US, Seiten 1383-1385, XP000461836 PLOTKIN S.A.: "Vaccines for Varicella-Zoster Virus and Cytomegalovirus: Recent Progress"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Gegenstand der vorliegenden Erfindung sind Arzneimittel enthaltend wenigstens ein Polypeptid bzw. Protein, das hinsichtlich der Aminosäuresequenz zumindest teilweise dem UL84-Protein des humanen Cytomegalovirus entspricht. Diese Polypeptide können zur Hemmung der Vermehrung von humanen Cytomegaloviren (im folgenden kurz HCMV) eingesetzt werden, wobei entweder die Polypeptide auf üblichem Wege verabreicht werden oder die Gene kodierend für diese Polypeptide mit Hilfe geeigneter Vektoren in die Zielzellen eingebracht werden und dort zur Expression gebracht werden.

Das humane Cytomegalovirus (HCMV) ist ein ubiquitär verbreitetes Virus. Es dürften etwa 50 % der Bevölkerung in Mitteleuropa als seropositiv angesehen werden und diese Personen tragen demnach das Virus in einer latenten Form in sich.

Das HCMV verursacht ein der Infektiösen Mononukleose ähnliches Krankheitsbild, aber ansonsten leiden gesunde Personen selten an einer HCMV-induzierten Erkrankung.
Bestimmte Risikogruppen, wie beispielsweise Neugeborene und vor allem immunsupprimierte Patienten, sowie die Empfänger von Transplantaten können durch HCMV-Infektionen bedroht sein. Es kann zu lebensbedrohlichen oder auch zu Erblindung führenden Krankheitsbildern wie Sepsis, Pneumonie, Colitis oder Retinitis kommen. Diese Komplikationen treten vor allem nach Organtransplantationen, bei HIV-Infektionen, unter Zytostatika-Therapie und bei Neugeborenen nach pränataler Infektion auf.

Die klinischen Manifestationen der HCMV-Infektion sind sehr vielfältig. Einerseits kann eine generalisierte Infektion im Sinn einer Sepsis entstehen, häufig ist aber das Krankheitsgeschehen auf ein Organsystem konzentriert, so daß eine lokalisierte therapeutische Behandlung wünschenswert ist. Zu den wichtigsten Organmanifestationen zählen die Pneumonie, gastrointestinale Erkrankungen (z.B. Gastritis, Ulzerationen im Magen/Darmtrakt), die Retinitis, Hautulzerationen (insbesondere bei AIDS-Patienten) sowie stenosierende Gefäßprozesse (z.B. nach perkutaner, transluminaler Koronarangioplastie = PTCA, nach Herztransplantation).

Bei vielen dieser Krankheitsbilder ist eine dauernde, virostatische Therapie notwendig, so daß hier ein Therapieprinzip, das zu einer "intrazellulären Immunität" gegen HCMV führt, von großem Vorteil ist.

Derzeit stehen zur Therapie von HCMV-Infektionen im wesentlichen zwei Medikamente zur Verfugung, nämlich Ganciclovir und Foscamet. Diese beiden Substanzen greifen an der viral kodierten DNA-Polymerase an, deren Funktion sie hemmen. Diese Substanzen haben aber gravierende Nachteile. Sie weisen ein ausgeprägtes Toxizitätsprofil auf, so daß bei 20 bis 40 % aller behandelten Patienten ein Abbruch der Therapie notwendig wird. Andererseits handelt es sich um eine virostatische Therapie, die zum Teil über lange Behandlungszeiträume erforderlich ist. Es kommt daher sehr leicht zum Auftreten von resistenten Virusvarianten und dadurch ist die Wirksamkeit dieser Medikamente nicht mehr gegeben.

Im Rahmen der vorliegenden Erfindung werden Polypeptide eingesetzt, die in die genetische Regulation des Cytomegalovirus eingreifen.

Wie allgemein bei Herpesviren, wird der Replikationszyklus des HCMV in drei verschiedene Phasen unterteilt, die als immediate early (sehr früh), early (früh) und late (spät) bezeichnet werden. Unmittelbar nach Eintritt des Virus in die Zielzelle kommt es zur Expression einer limitierten Anzahl von Proteinen, den sogenannten immediate early-Proteinen, die immunologisch klassifiziert werden können. Diese immediate early-Proteine haben zum Teil eine Transaktivator-Funktion, sie können also andere virale Promotoren aktivieren und leiten somit die nächste Phase der viralen Genexpression, also die early oder Frühphase ein. Gleichzeitig modifizieren einige dieser immediate early-Proteine auch die zelluläre Genexpression und können heterologe virale Promotoren beeinflussen.

Essentiell für eine Aktivierung von frühen viralen Promotoren ist hierbei das IE2-Protein, das eine Molekularmasse von 86 kDa aufweist und in der Literatur auch als IE86, IE80 oder IE2-86 bezeichnet wird. Das IE86-Protein ist notwendig für die Stimulation der frühen Genexpression, während andere IE-Proteine einen zusätzlichen, verstärkenden Effekt ausüben können.

Im Rahmen der vorliegenden Erfindung wurde festgestellt, daß ein anderes von HCMV stammendes Protein, das Protein UL84, einerseits die transaktivierende Funktion von IE86 hemmt und andererseits die durch IE86 vermittelte Negativ-Regulation des IE1/2 Enhancer/Promotor verstärkt. Aufgrund dieser Erkenntnis konnte nachgewiesen werden, daß eine Expression des Proteins UL84 während der immediate early-Phase des Replikationszyklus von HCMV die Virusvermehrung hemmt.

Im Rahmen der vorliegenden Erfindung konnte die Interaktion des IE86-Proteins mit dem viral kodierten UL84-Protein abgeklärt werden. Hierzu wurde zunächst ein eukaryonter Expressionsvektor konstruiert, der das UL84-Protein des HCMV fusioniert mit einem antigenen Peptid (FLAG_-Epitop) exprimierte. Dies erlaubte den immunologischen Nachweis des Proteins mit Hilfe eines gegen das FLAG_-Epitop gerichteten monoklonalen Antikörpers. COS7-Zellen wurden kotransfiziert einerseits mit diesem UL84-Expressionsvektor und andererseits mit einem Vektor, der das IE86-Protein exprimierte. Eine anschließende Immunpräzipitationsanalyse zeigte, daß UL84, unabhängig von anderen viralen Proteinen, eine stabile Interaktion mit IE86 eingehen kann. Dies wurde auch durch einen in vitro-Versuchsansatz, eine sogenannte "pull down"-Analyse bestätigt.

Um die funktionellen Effekte dieser Interaktion zu untersuchen, wurden Reporterkonstrukte, die Luziferase unter Kontrolle verschiedener homologer und heterologer Promotoren exprimierten, zusammen mit den UL84- und/oder IE86-Expressionsplasmiden in Kotransfektionsexperimenten eingesetzt. Diese Versuche ergaben den überraschenden Befund, daß das UL84-Protein die IE86-vermittelte Transaktivierung sehr stark und dosisabhängig hemmte, während es für sich allein genommen die untersuchten Promotoren nicht beeinflußte. Dies konnte sowohl für den Promotor des menschlichen Immundefizienzvirus als auch für einen frühen viralen Promotor des HCMV, nämlich den UL112-Promotor nachgewiesen werden.

Nachdem die Aktivierung von frühen viralen Promotoren essentiell ist für ein Fortschreiten des Replikationszyklus, ergab sich im Rahmen der vorliegenden Erfindung, daß eine Expression von UL84 zu immediate early-Zeiten die Replikation des HCMV hemmen kann. Dies konnte durch zwei unterschiedliche Versuchsansätze bestätigt werden. Nach Transfektion von für HCMV permissiven U373-Zellen mit dem das Protein UL84 exprimierenden Plasmid und anschließender Superinfektion mit HCMV konnte in UL84 exprimierenden Zellen kein frühes virales Antigen nachgewiesen werden. Dies ist ein klarer Hinweis auf die Hemmung der frühen Genexpression durch UL84.

In einem anderen Versuchsansatz wurden Zellinien etabliert, die das UL84-Protein stabil exprimierten. Nach Infektion dieser Zellen mit HCMV konnte zwar noch immediate early-Protein nachgewiesen werden; dies zeigt, daß die Zellen prinzipiell infizierbar sind. Es kam allerdings nicht zur Expression von early-Proteinen. Gleichzeitig konnten weder die für HCMV typischen morphologischen Veränderungen der Zellen beobachtet werden, noch kam es zu einer Zerstörung der infizierten Zellen. Eine Expression von UL84 zu Beginn der Infektion kann damit die Replikation von HCMV effizient hemmen. Dies ist auf eine Neutralisierung der transaktivierenden Wirkung des IE86-Proteins zurückzuführen, die durch eine spezifische und hochaffine Protein/Protein-Interaktion vermittelt wird.

Aufgrund der im Rahmen der vorliegenden Erfindung gewonnenen Erkenntnisse ist es daher möglich, Arzneimittel zur Verfügung zu stellen, die Polypeptide aufweisen, die hinsichtlich der Aminosäuresequenz dem pUL84-Protein zumindest teilweise entsprechen. Im Rahmen der vorliegenden Erfindung wurde gefunden, daß der N-terminale Bereich des UL84-Proteins für die Bindung an den IE 1/2 Enhancer/Promotor notwendig ist und daher bei diesen Arzneimitteln in bevorzugter Ausführungsform vorhanden ist.

Alternativ hierzu kann das für das UL84-Protein kodierende Gen auch mit Hilfe eines Vektors in die Zellen verbracht werden und dort zur Expression gebracht werden. Derartige Vektoren enthalten dann wenigstens einen Teil des für UL84 kodierenden Gens. Gegenstand der vorliegenden Erfindung sind daher Arzneimittel, die ein Polypeptid aufweisen, das zumindest einen Teil der Aminosäuresequenz des Proteins UL84 des Cytomegalovirus oder ein dazu homologes Polypeptid umfassen, wobei eine Homologie von wenigstens 80 % vorliegt.

Bevorzugt weisen diese Arzneimittel ein Polypeptid auf, dessen Aminosäuresequenz dem Protein UL84 wenigstens zum Teil entspricht, wobei diese Aminosäuresequenz in Abbildung 1 dargestellt ist.

Die erfindungsgemäßen pharmazeutischen Zubereitungen weisen bevorzugt Polypeptide auf, deren Aminosäuresequenz wenigstens dem N-terminalen Bereich des UL84-Proteins entspricht. In besonders bevorzugter Ausführungsform werden wenigstens die 180 N-terminalen Aminosäuren des UL84-Proteins umfaßt

Die erfindungsgemäßen pharmazeutischen Zubereitungen dienen bevorzugt der Behandlung einer Infektion von humanem Cytomegalovirus.

Dementsprechend können die erfindungsgemäß verwendeten Polypeptide, die zumindest einen Teil der Aminosäuresequenz des UL84-Proteins des humanen Cytomegalovirus umfassen, oder dafür kodierende Nucleinsäuren zur Therapie oder Prophylaxe einer Infektion von humanem Cytomegalovirus eingesetzt werden.

Die erfindungsgemäß verwendeten Polypeptide bzw. die dafür kodierenden Nucleinsäuren können in Verfahren eingesetzt werden, bei denen ein für wenigstens einen Teil des Proteins UL84 kodierendes Gen mit Hilfe geeigneter Vektoren in die gewünschten Zielzellen eingeführt wird und dann das UL84-Protein in dieser Zelle exprimiert wird. Derartige Verfahren dienen der Verhinderung der Vermehrung von humanen Cytomegaloviren.

Bei den eingesetzten Arzneimitteln kann es sich um solche handeln, die mit Hilfe herkömmlicher Methoden formuliert werden.

Wichtig ist, daß das Polypeptid in unveränderter Form an die Zielzellen gelangt und dann in die Zielzellen eindringen kann. Sofern orale Verabreichungsformen gewählt werden, muß sichergestellt werden, daß das Arzneimittel die Magen-Darm-Passage unverändert überstehen kann.

Das UL84-Protein ist an sich bekannt, da sowohl die Nucleinsäure- wie auch die Aminosäuresequenz vorpubliziert wurde. In der Abbildung 1 ist die Nucleinsäure- und Aminosäuresequenz von UL84 dargestellt.

Da es sich bei dem erfindungsgemäß eingesetzten UL84-Protein um ein virales Protein handelt, umfaßt die vorliegende Erfindung auch zu UL84 homologe Polypeptide. Die Homologie stellt eine Bezeichnung für den Grad der Übereinstimmung von zwei Proteinsequenzen dar. 50 % Homologie bedeutet beispielsweise, daß 50 von 100 Aminosäurepositionen in der Sequenz übereinstimmen. Im vorliegenden Fall werden von der Erfindung auch solche Proteine umfaßt, die zu wenigstens 80 % homolog zu dem pUL84-Protein sind. Dies bedeutet, daß 8 von 10 Aminosäuren in den vergleichbaren Sequenzen übereinstimmen.

Da im Rahmen der vorliegenden Erfindung herausgefunden wurde, daß der N-terminale Bereich für die Bindung an den IE 1/2 Enhancer/Promotor notwendig ist, umfassen die erfindungsgemäßen Arzneimittel wenigstens diesen N-terminalen Bereich, wobei wenigstens etwa 180 terminale Aminosäuren des UL84-Proteins vorhanden sein sollten.

Es ist auch möglich, nicht das Polypeptid zur Therapie einzusetzen, sondern das für das UL84-Protein kodierende Gen bzw. Teilgen. Bei dieser Verwendung wird das Gen mit einem Expressionsvektor in die gewünschten Zielzellen gebracht und in den Zellen wird dann das UL84-Protein bzw. Teilpolypeptid exprimiert. Verwendet werden kann dies einerseits zur Therapie von bereits bestehenden HCMV-Infektionen und andererseits zur Prophylaxe gegenüber drohenden HCMV-Infektionen.

Die vorliegende Erfindung wird durch die anliegenden Abbildungen näher erläutert:

Abbildung 1 zeigt die Nucleinsäure- und davon abgeleitete Aminosäuresequenz des offenen Leserahmens für UL84 des humanen Cytomegalovirus, Stamm AD169.

Abbildung 2 stellt eine schematische Darstellung des eukaryonten Expressionsplasmids pcDNA-UL84 dar.

Abbildung 3 stellt den Nachweis einer von anderen viralen Proteinen unabhängigen Interaktion von pUL84 und EI86 durch Immunpräzipitation und "pull down"-Analyse dar. Bei der Abbildung bedeuten:

A) COS-7-Zellen wurden entweder mock-transfiziert oder mit den Expressionsplasmiden pHM121 (IE86) und/ oder pcDNAUL84 transfiziert. Nach radioaktiver Markierung von Zellproteinen wurden Zellysate hergestellt und durch Immunpräzipitation mit dem IE86-spezifischen, monoklonalen Antikörper 2.9.5 untersucht. M: Größenmarker; Spur 1: Lysat aus mock-transfizierten Zellen; Spur 2, Lysat aus Zellen, die mit pHM121 transfiziert worden waren; Spur 3, Lysat aus Zellen, die mit pHM121 und pcDNAUL84 transfiziert worden waren.

B) GST-"Pull down"-Experiment: die Proteine TBP, IE1 und UL84 wurden durch *in vitro*-Translation radioaktiv markiert und dann entweder mit einem GST-IE1 oder GST-IE2 Fusionsprotein inkubiert. Nach extensivem Waschen der Agarose-gekoppelten GST-Proteine wurden gebundene Proteine durch Erhitzen auf 95 % denaturiert und durch SDS-PAGE analysiert. M: Größenmarker; Spuren 1-3, die *in vitro*-translatierten Proteine TBP, IE1 und UL84, die für die Bindungsreaktion verwendet wurden; Spuren 4-6, TBP, IE1 und UL84 nach Inkubation mit GST-IE1; Spuren 7-9, TBP, IE1 und UL84 nach Bindung an GST-IE2.

Abbildung 4 zeigt den Einfluß von UL84 auf die IE2-vermittelte Transaktivierung homologer und heterologer viraler Promotoren.
U373-Zellen wurden mit Reporterplasmiden, die verschiedene Promotoren oberhalb von Luciferase enthielten, und Expressionsplasmiden für IE86 (pHM134), pUL84 (pcDNAUL84) oder das HIV tat-Protein (pCT21) kotransfiziert. Nach 48 h wurden die Zellen lysiert und die Luciferaseaktivität bestimmt. In der Abbildung ist die Aktivierung (als x-fache Aktivierung) nach Kotransfektion verschiedener Expressionsplasmide relativ zur Basisaktivität des Promotors (Aktivität des Promotors nach Kotransfektion des Klonierungsvektors pcDNA3) dargestellt. A) Als Reporterplasmid wurden 3 μg eines HIV-Promotor-Luciferasekonstrukts verwendet. B) Als Reporterplasmid wurden 3 μg eines UL112-Promotor-Luciferasekonstrukts verwendet. Spuren: 1, Kotransfektion mit 10 μg pcDNA3; 2, Kotransfektion mit 5 μg pcDNAUL84 und 5 μg pcDNA3; 3, Kotransfektion mit 5 μg pHM134; 4, Kotransfektion mit 5 μg pHM134 und 5 μg pcDNAUL84; 5, Kotransfektion mit 5 μg pCT21 und 5 μg pcDNA3; 6, Kotransfektion mit 5 μg pCT21 und 5 μg pcDNAUL84. C) Als Reporterplasmid wurden 3 μg eines UL112-Promotor-Luciferasekonstrukts verwendet. Spuren 1, Kotransfektion mit 10 μg pcDNAUL84; 2, Kotransfektion mit 10 μg pcDNAUL84; 3, Kotransfektion mit 3 μg pHM134 und 7 μg pcDNA3; 4, Kotransfektion mit 3 μg pHM134, 1 μg pcDNAUL84 und 6 μg pcDNA3; 5, Kotransfektion mit 3 μg pHM134, 2 μg pcDNAUL84 und 5 μg pcDNA3; 6, Kotransfektion mit 3 μg pHM134, 3 μg pcDNAUL84 und 4 μg pcDNA3; 7, Kotransfektion mit 3 μg pHM134, 4 μg pcDNAUL84 und 3 μg pcDNA; 8, Kotransfektion mit 3 μg pHM134, 5 μg pcDNAUL84 und 2 μg pcDNA3; 9, Kotransfektion mit 3 μg pHM134, 6 μg pcDNAUL84 und 1 μg pcDNA3; 10, Kotransfektion mit 3 μg pHM134 und 7 μg pcDNAUL84. D) Als Reporterkonstrukt wurden 3 μg eines IE-1/2 Enhancer/Promotor-Luceferasekonstrukts verwendet. Spuren: 1, Kotransfektion mit 10 μg pcDNA3; 2, Kotransfektion mit 5 μg pcDNA3 und 5 μg pcDNAUL84; 3, Kotransfektion mit 5 μg pcDNA3 und 5 μg pHM134; 4, Kotransfektion mit 5 μg pHM134 und 5 μg pcDNAUL84.

Abbildung 5 zeigt die Hemmung der HCMV-Replikation nach transienter Expression von pUL84.
U373-Zellen wurden mit dem Expressionsplasmid pcDNAUL84 transfiziert und mit HCMV infiziert. Anschließend wurde die Expression von pUL84 und des frühen viralen Antigen pUL69 durch Doppel-Immunfluoreszenzanalyse unter Verwendung eines konfokalen Lasermikroskops untersucht. Dies zeigte, daß in pUL84-exprimierenden Zellen nie gleichzeitig pUL69 synthetisiert wurde. A) Nachweis von pUL84. B) Nachweis von pUL69. C) Überlagerung der Abbildungen A und B.

Abbildung 6 zeigt die Hemmung der frühen viralen Genexpression nach stabiler Expression von pUL84.
Es wurden U373-Zellinien etabliert, die pUL84 stabil exprimierten. Diese Zellinien wurden mit HCMV infiziert und anschließend die Proteinexpression durch indirekte Immunfluoreszenz analysiert. A bis E, Zellinie IXB1, wurde mit pcDNAUL84 transfiziert und exprimiert pUL84; B bis F, Zellinie DC1, wurde mit dem Klonierungsvektor pcDNA3 transfiziert, exprimiert kein pUL84. A und B, Nachweis von pUL84 mit Hilfe des monoklonalen Antikörpers M2; C und D, Nachweis von *immediate early* Antigen mit Hilfe eines monoklonalen Antikörpers gegen das IE1-Protein; E und F, Nachweis von frühem Antigen mit Hilfe eines Antiserums gegen das UL69-Protein.

Abbildung 7 zeigt die Hemmung der HCMV-Replikation nach stabiler Expression von UL84.
U373-Zellen, die entweder UL84-negativ waren (A bis C) oder pUL84 stabil exprimierten (D bis F) wurden mock-infiziert (A und D) oder mit HCMV mit einer M.O.I. von 2 infiziert (B, C, E, F). B und E zeigen den cytopathogenen Effekt nach 5 Tagen Infektionsdauer, C und F nach 23 Tagen Infektionsdauer.

Abbildung 8 zeigt die Hemmung der Virusvermehrung nach Infektion von UL84-exprimierenden Zellinien mit HCMV.

Die UL84-negative Zellinie pRcCMVDC1 und die UL84-positiven Linien ULS4IXC5 und ULS4IXB6 wurden mit HCMV mit einer M.O.I von 0,1 infiziert. Zu verschiedenen Zeitpunkten nach Infektion (0, 3, 4, 7, 10 und 12 Tage) wurde Zellkulturüberstand entnommen und eine Virustitration durch Bestimmung der "Tissue Culture Infectious Dose" ($TCID_{50}$) durchgeführt.

**Beispiel 1**

**Konstruktion eines eukaryonten Expressionsvektors für pUL84**

Zur Untersuchung der Eigenschaften von pUL84 wurde der offene Leserahmen UL84 (Abb. 1) unter Verwendung der Primer UL845' (TAAGAATTCATGCCACGCGTCGACCCCAACCTTCGGAAT) und UL843' (TAATCTAGATCCCTAGGTACCTTCGAGATCGCCGCAGACCATGGCTAAAGTGA C) mit Hilfe der Polymerase-Kettenreaktion amplifiziert und über die in den Primem kodierten *Eco*RI und *Xba*I Schnittstellen in den Vektor pSG424 (Sadowski, I. and M. Ptashne, 1989, A vector for expressing GAL4(1-147) fusions in mammalian cells, Nucleic. Acids. Research 17:7539) kloniert. Zur Überprüfung der Expression von pUL84 nach Transfektion wurde durch chemische Synthese ein DNA-Fragment hergestellt, welches für ein Oktapeptid kodiert ($FLAG^R$) und anschließend über *Kpn*I und *Xba*I in dieses Plasmid kloniert (Fig. 2); dieses Peptid wird durch monoklonale Antikörper erkannt und kann als sogenanntes TAG in Fusion mit heterologen Proteinen dazu verwendet werden, die Expression des heterologen Proteinanteils in eukaryonten Zellen zu überprüfen. Nach Transfektion des resultierenden Plasmides pSG84TAG in COS7-Zellen konnte mit Hilfe des Antikörpers M2 (Fa. Integra Biosciences; Tecnomara Deutschland GmbH) die Expression des pUL84::FLAG Fusionsproteins nachgewiesen werden. Zur Verstärkung der Expression wurde anschließend die Expressionskassette bestehend aus der kodierenden Region für pUL84::FLAG über Spaltung mit *Eco*RI und XbaI in den Vektor pcDNA3 (Abb. 2) umkloniert. Das resultierende Plasmid pcDNAUL84 wurde für die weiteren Experimente eingesetzt.

**Beispiel 2**

**Nachweis einer spezifischen Interaktion des UL84-Proteins mit dem IE86-Transaktivator**

Um zu überprüfen, ob pUL84 unabhängig von anderen viralen Proteinen eine spezifische Interaktion mit dem IE86-Transaktivatorprotein des HCMV eingehen kann, wurden COS7-Zellen entweder nur mit dem IE86-Expressionsplasmid pHM121 (Plachter et al. (1993) Analysis of proteins encoded by IE-regions 1 and 2 of human cytomegalovirus using monoclonal antibodies generated against recombinant antigens, Virology 193:642-652) oder mit einer Kombination aus pHM121 und pcDNAUL84 transfiziert. Die Transfektion erfolgte mit Hilfe der DEAE-Dextran-Methode unter Verwendung von je 10 μg Vektor-DNA (Winkler et al. (1994) UL69 of human cytomegalovirus, an open reading frame with homology to ICP27 of herpes simplex virus, encodes a transactivator of gene expression, J. Virol. **68**:3943-3954). Anschließend wurden die Zellproteine durch Zugabe von "Tran-$^{35}$S-Label" (ICN, Eschwege) radioaktiv markiert und durch Immunpräzipitation analysiert. Die Zellen wurden dazu von der Zellkulturschale abgelöst, sedimentiert und durch Resuspendieren und einstündige Inkubation in 1 ml eines Puffers aus 50 mM Tris/HCl, pH 8.0, 5 mM EDTA, 150 mM NaCl, 0.5% NP-40, 1 mM PMSF und 20 mg/ml Aprotinin lysiert. Je 250 μl des Lysats wurden für 3 h bei 4°C mit Protein-A-Sepharose (Sigma, Deisenhofen) inkubiert und dann bei 4000 upm zur Entfernung unspezifisch adsorbierender Substanzen zentrifugiert. Der IE86-spezifische monoklonale Antikörper 2.9.5 (Plachter et al. (1993) Analysis of proteins encoded by IE-regions 1 and 2 of human cytomegalovirus using monoclonal antibodies generated against recombinant antigens, Virology 193:642-652) wurde durch Inkubation bei 4°C in Bindepuffer (100 mM Tris/HCl, pH 7.4, 400 mM NaCl) zusammen mit Anti-Maus IgG an Protein A-Sepharose gekoppelt. Der Protein A Sepharose-Antikörperkomplex wurde dann mit den radioaktiv markierten Proteinen gemischt und 3 h bei 4°C inkubiert. Nach mehreren Wasch-Schritten unter Verwendung von SNNTE-Puffer (50 mM Tris/HCl, pH 7.4, 5 mM EDTA, 100 mM NaCl, 5% Sucrose, 1% NP40) wurde der Komplex in SDS-haltigem Probenpuffer für die Polyacrylamid-Gelelektrophorese (PAGE) aufgenommen und durch SDS-PAGE und Autoradiographie analysiert (Abb. 3). Nach Transfektion des IE86-Expressionsplasmids pHM121 präzipitierte der Antikörper 2.9.5 ein Protein mit einer Molekularmasse von 86 kDa, das in mock-transfizierten Zellen nicht vorhanden war (Abb. 3, A, Spuren 1 und 2). Wurden die Zellen jedoch mit den Plasmiden pHM121 und pcDNAUL84 kotransfiziert, so konnte ein weiteres Protein nachgewiesen werden, das pUL84 entsprach (Abb. 3, A, Spur 3). Dies zeigt, daß pUL84 unabhängig von weiteren viralen Proteinen einen stabilen Komplex mit IE86 eingehen kann.

Um dies zusätzlich abzusichern und zu beweisen, daß diese Interaktion unabhängig ist von posttranslationalen Modifikationen des IE86-Proteins, wurde eine "pull down"-Analyse durchgeführt. Dazu wurden die Proteine IE86 und IE1 des HCMV als Fusionen mit Glutathion-S-Transferase (GST) prokaryont exprimiert und durch Kopplung an Glutathion-Agarose (Pharmacia, Freiburg) gereinigt (Lang et al. (1995) Functional interaction between the human cytomegalovirus 86-kilodalton IE2 protein and the cellular transcription factor CREB, J. Virol., 69: pp. 6030-6037). Parallel dazu wurden das TATA-bindende Protein TBP, das IE1-Protein des HCMV und das UL84-Protein unter Verwendung des TNT-Systems (Promega, Heidelberg) durch in vitro-Translation unter Zugabe von $^{35}$S-Methionin radioaktiv markiert. Die GST-Fusionsproteine (je 100 ng, an Glutathion-Agarose gekoppelt) wurden für 10 min in 200 ml ELB-Puffer (125 mM NaCl, 50 mM HEPES, pH 7.0, 0.1 % NP-40, 1 mM PMSF, 0.5 mM DTT, 0.5 mM EDTA) mit 1 mg/ml bovinem Serumalbumin inkubiert. Dann wurden je 5 μl der *in vitro*-translatierten Testproteine zugegeben. Nach einer weiteren Inkubation für 12 h bei 4°C wurden die Agarose-gekoppelten Proteine fünfmal mit je 1 ml ELB-Puffer gewaschen, in SDS-Probenpuffer aufgenommen, auf 95°C erhitzt und durch SDS-PAGE analysiert. Die Autoradiographie ergab, daß das in vitro-translatierte UL84-Protein an GST-IE2 gebunden hatte, während keine Interaktion mit dem als Spezifitätskontrolle verwendeten GST-IE1 zu beobachten war (Abb. 3, B, Spuren 6 und 9). pUL84 interagierte mit GST-IE2 mit etwa gleicher Effizienz wie das TATA-bindende Protein TBP, für das schon in vorhergehenden Studien eine starke Bindung an IE2 gezeigt worden war (Abb. 3, B, Spuren 7 und 9). pUL84 kann somit unabhängig von post-translationalen Modifikationen mit dem IE2-Protein interagieren. Weitere "pull down"-Experimente mit carboxy-terminal verkürzten UL84-Proteinen zeigten, daß eine Deletionsmutante, die durch Spaltung mit *Pvu*I hergestellt worden war, noch effizient an GST-IE2 band. Eine Interaktionsdomäne von pUL84 befindet sich demnach in den aminoterminalen 180 Aminosäuren (AS) des insgesamt 586 AS umfassenden Proteins.

**Beispiel 3**

**Hemmung der IE86-vermittelten Transaktivierung durch pUL84**

Das IE86-Protein ist ein starker Transaktivator von homologen und heterologen Promotoren und in dieser Funktion essentiell für die Einleitung der frühen Phase der viralen Genexpression. Um die funktionellen Auswirkungen der Interaktion von IE86 mit pUL84 zu untersuchen, wurden transiente Expressionsanalysen unter Verwendung der für HCMV permissiven Zellinie U373 MG durchgeführt. Als Reporter wurde das Luciferasegen verwendet, vor das verschiedene Promotoren, wie der Promotor des menschlichen Immundefizienzvirus (HIV-Promotor), der frühe UL112-Promotor des HCMV oder der IE-1/2 Enhancer/Promotor des HCMV kloniert waren. Je 3 μg dieser Reporterplasmide wurden mit entweder je 5 μg pcDNAUL84 oder pHM134 (IE86-Expressionsplasmid) allein oder einer Kombination beider Vektoren kotransfiziert (Lang et al. (1995) Functional interaction between the human cytomegalovirus 86-kilodalton IE2 protein and the cellular transcription factor CREB, J. Virol., in press). Die insgesamt transfizierte DNA-Menge wurde durch Zugabe von DNA des Klonierungsvektors pcDNA3 bis zu einer DNA-Menge des Transfektionsansatzes von 15 μg konstant gehalten. Die Transfektion erfolgte unter Verwendung der DEAE-Dextran-Methode (Arit et al. (1994) Identification of binding sites for the 86-kilodalton IE2 protein of human cytomegalovirus within an IE2-responsive viral early promoter, J. Virol. **68**:4117-4125). Nach 48 Stunden Inkubation wurden die Zellen in 1 ml Extraktionspuffer (100 mM Kaliumphosphat, pH7.8, 1 mM DTT) geerntet und durch dreimaliges Einfrieren/Auftauen lysiert. Durch Zentrifugation wurde unlöslicher Zelldetritus abgetrennt. Gleiche Mengen des Überstands wurden dann mit 100 μl Reaktionspuffer (100 mM Kaliumphosphat, pH7.8, 15 mM $MgSO_4$, 5 mM ATP) gemischt und die Luciferaseaktivität durch Injektion von 100 μl Reaktionspuffers mit 1 mM Luciferin (Boehringer, Mannheim) unter Verwendung eines Luminometers (Berthold, Wildbad) bestimmt. Jede Transfektion wurde wenigstens dreimal wiederholt. Wurde das HIV-Promotorkonstukt mit pcDNAUL84 allein kotransfiziert, so konnte keine veränderte Aktivität im Vergleich zur Transfektion des Klonierungsvektors pcDNA3 beobachtet werden; pUL84 allein übt demnach keinen Effekt auf diesen Promotor aus (Abb. 4, A, Spuren 1 und 2). Die Kotransfektion des IE86-Expressionsplasmids pHM134 führte zu einer etwa 60fachen Aktivierung dieses Promotors (Abb. 4, A, Spur 3). Wurde jedoch eine Kombination von pcDNAUL84 und pHM137 eingesetzt, so kam es zu einer starken Reduktion der durch IE86-vermittelten Transaktivierung des HIV-Promotors (Abb. 4, A, Spur 4). Dieser Negativ-Effekt von UL84 war spezifisch, da die Transaktivierung durch den homologen Transaktivator des HIV, das tat-Protein, nicht durch Kotransfektion von pcDNAUL84 beeinflußt wurde (Abb. 4, A, Spuren 5 und 6). Negativ-Regulation der IE86-Transaktivierung ließ sich nicht nur am HIV-Promotor beobachten, sondern auch an einem frühen Promotor des HCMV, dem UL112-Promotor; auch hier führte die Kotransfektion von pcDNAUL84 zusammen mit pHM137 zu einer drastischen Reduktion der Aktivierung, die mit pHM137 allein zu sehen war (Abb. 4, B, Spuren 3 und 4). Dieser Effekt erwies sich als dosis-abhängig: wurden steigende Mengen an pcDNA-UL84 mit einer konstanten Menge pHM137 verwendet, so kam es zu einer deutlichen Verstärkung des Negativ-Effekts (Abb. 4, C, Spuren 4 bis 10). Neben einer transaktivierenden Wirkung hat das IE86-Protein auch eine negativ-autoregulatorische Funktion: es kann seine eigene Expression reprimieren, indem es die Aktivtät des dafür verantwortlichen Promotors, des IE-1/2 Enhancer/Promotors, negativ beeinflußt (Pizzorno et al. (1990) The IE2 gene products of human cytomegalovirus specirically down-regulate expression from the major immediate-early promoter through a target sequence located near the cap site, J. Virol. **64**:6154-6165). Wurde dieser Promotor als Fusion mit Luciferase in Kotransfektionsexperimenten verwendet, so zeigte sich, daß pUL84 die durch IE86 bewirkte Repression dieses Promotors nicht aufhob, sondern sogar verstärkte (Abb. 4, D, Spuren 3 und 4). pUL84 neutralisiert damit ganz spezifisch die transaktivierende Funktion von IE86 während die negativ-autoregulatorische Funktion erhalten bleibt bzw. sogar verstärkt wird.

**Beispiel 4**

**Hemmung der HCMV-Replikation nach transienter Expression von pUL84**

Aufgrund der oben genannten Befunde ergab sich die Hypothese, daß eine Expression von pUL84 zu Beginn des Replikationszyklus zu einer Aufhebung der transaktivierenden Wirkung von IE86 und damit zu einer Hemmung der HCMV-Replikation führen müßte. Dies wurde zunächst durch transiente Expression von pUL84, Überinfektion mit HCMV und anschließender Immunfluoreszenzanalyse der Proteinexpression untersucht. U373-Zellen wurden mit 10 μg pcDNAUL84 unter Verwendung der Calciumphosphat-Kopräzipitationsmethode transfiziert. Etwa 24 h später wurden die Zellen mit HCMV mit einer M.O.I. (multiplicity of infection) von 2 infiziert. Nach weiteren 60 h wurden die Zellen durch 10minütige Inkubation in eiskaltem Methanol fixiert. Es folgte dann eine Doppelfärbung der Zellen für die Immunfluoreszenz-Analyse: die Expression des transient exprimierten pUL84 ließ sich durch den gegen das FLAGR-TAG gerichteten Maus-monoklonalen Antikörper M2 (Integra BioSciences, Tecnomara Deutschland GmbH) nachweisen; die frühe virale Genexpression wurde durch ein im Kaninchen hergestelltes Antiserum gegen das früh-spät exprimierte UL69-Protein analysiert (Winkler et al. (1994) UL69 of human cytomegalovirus, an open reading frame with homology to ICP27 of herpes simplex virus, encodes a transactivator of gene expression, J. Virol. 68:3943-3954). Die Antikörper wurden in PBSo-Puffer (138 mM NaCl, 2.7 mM Kcl, 6.5 mM $Na_2HPO_4$, 1.5 mM $KH_2PO_4$) verdünnt (M2-Antikörper: 1:1000; UL69-Antiserum: 1:200) und für 30 min bei 37°C mit den Zellen inkubiert. Nach dreimaligem Waschen mit $PBSO_4$ wurden ein Rhodamin (TRITC)-konjugierter Ziege-Anti-Maus-Antikörper (Dianova, Hamburg) und ein Fluorescein (FITC)-konjugierter Schwein-Anti-Hase-Antikörper (Dako, Glostrup, Dänemark) je 1:40 in PBSo verdünnt und wiederum 30 min bei 37° zusammen mit den Zellen inkubiert. Nach drei weiteren Wasch-Schritten wurden die Zellen eingedeckt und spezifisch gefärbte Zellen mit Hilfe der konfokalen Lasermikroskopie unter Verwendung des Systems MRC600 der Firma Bio-Rad, München, analysiert. Hierbei wurden zunächst Rhodamin- (rote Färbung, spezifisch für UL84) und Fluorescein- (grüne Färbung, spezifisch für UL69) gefärbte Zellen separat nachgewiesen (Abb. 5, A, B) und anschließend die beiden Fluoreszenzmuster übereinandergelagert (Abb. 5, C). Wie exemplarisch in Figur 5 zu sehen ist, zeigte dieses Experiment, daß in Zellen, die UL84 synthetisierten, das früh exprimierte UL69-Antigen nicht nachweisbar war. Dies war der erste Hinweis auf eine Hemmung der HCMV-Replikation durch Expression von pUL84.

**Beispiel 5**

**Hemmung der HCMV-Replikation nach stabiler Expression von pUL84**

Um den hemmenden Effekt von pUL84 auf die HCMV-Replikation weiter abzuklären, wurden U373-MG-Zellinien etabliert, die pUL84 stabil exprimierten. Hierzu wurden U373-Zellen mit 10 μg des Vektors pcDNAUL84 oder des Klonierungsvektors pcDNA3, die beide das Neo-Gen als Selektionsmarker enthielten, durch Calciumphosphat-Kopräzipitation transfiziert. Etwa 48 h nach Transfektion wurde Geneticin (500 μg/ml) zum Zellkulturmedium zugegeben. Nach etwa 4 Wochen Selektionsdauer hatten sich Zellkione gebildet, die isoliert und in 24-Loch-Platten subkultiviert wurden. Anschließend wurde mit Hilfe der indirekten Immunfluoreszenz und des monoklonalen Antikörpers M2 die pUL84-Genexpression dieser Zellklone überprüft. Dadurch gelang es, 10 Zellinien zu etablieren, die pUL84 mit unterschiedlicher Effizienz exprimierten. Stark exprimierende Zellinien wurden für Infektionsversuche eingesetzt: dazu wurden pUL84-exprimierende Zellinien und parallel selektionierte Linien, die kein UL84 synthetisierten, mit HCMV mit einer M.O.I von 2 infiziert. Etwa 60 h nach Infektion wurden die Zellen durch Methanolbehandlung fixiert und durch indirekte Immunfluoreszenz analysiert. Wie in Abb. 6 exemplarisch für zwei Zellinien gezeigt ist, ließ sich in der Linie IXB1 mit Hilfe des monoklonalen Antikörpers M2 eine deutliche UL84-Expression nachweisen, während DC1 negativ war (Abb. 6, A, B). Die Anfärbung der Zellen mit einem monoklonalen Antikörper gegen das *immediate early*-Antigen IE1 des HCMV ergab mit beiden Zellinien eine starke Reaktivität; dies zeigte, daß HCMV beide Zellinien infiziert hatte (Abb. 6, C, D). Wurde jedoch jetzt das Antiserum gegen das früh-spät exprimierte UL69-Protein verwendet, so konnten in der UL84-negativen DC1-Zellinie starke Signale beobachtet werden, während in der UL84-positiven Linie IXB1 keine signifikante Antigenexpression vorhanden war. Damit war in IXB1 durch die UL84-Expression der Replikationszyklus auf Ebene der *immediate early*-Genexpression arretiert. Dies konnte auch bei längerer Infektionsdauer bestätigt werden. Dazu wurden die UL84-positive Zellinie IXC5 und die negative Linie DC2 mit HCMV mit einer M.O.I von 2 infiziert und hinsichtlich ihrer morphologischen Veränderungen, die typisch sind für den Replikationszyklus des HCMV, beurteilt. Nach 5 Tagen Infektionsdauer konnte in der Linie DC2 eine starker cytopathogener Effekt (CPE) nachgewiesen werden, während die Linie IXC5 keine Veränderungen erkennen ließ (Abb. 7, B, E). Nach 23 Tagen Infektionsdauer waren nahezu alle Zellen der Linie DC1 lysiert; für IXC5 ließ sich nach wie vor kein CPE erkennen (Abb. 7, C, F).

Zur Analyse der Virusproduktion wurden die Linien DC1 (keine UL84 Expression), sowie IXB6 und IXC5 (UL84-Expression) mit HCMV mit einer Multiplizität der Infektion von 0,1/Zelle infiziert. Nach unterschiedlichen Zeiten nach Infektion wurde Zellkultur-Überstand entnommen und bis zur Analyse bei -80 C weggefroren. Zur Bestimmung der sog. "*Tissue Culture Infectious Dose*" *50 (TCID50)* wurden humane Vorhaut-Fibroblasten auf 96-Loch-Platten ausgesät ($2x10^4$ Zellen pro Loch). Am nächsten Tag, nach subkonfluentem Anwachsen der Zellen, wurden Verdünnungsreihen der einzelnen infektiösen Überstände hergestellt. ($10^{-1}$ -$10^{-9}$ in 10er Schritten) und jeweils in Vierfach-Ansätzen zur Infektion der Zellen verwendet. Nach 24-stündiger Inkubation bei 37°C wurden die Zellen zur weiteren Analyse mit Methanol (-20°C/10 Min.) fixiert. Zum Nachweis der Expression des *immediate-early* Proteins IE1-pp72 wurde Kulturüberstand des monoklonalen Antikörpers BS500 (Biotest) unverdünnt zu den Zellen gegeben und für 45 Min. bei 37°C in einer feuchten Kammer inkubiert. Nach mehrfachen Waschschritten in PBS wurde zum Nachweis der spezifischen Antigen-Antikörperreaktion ein sekundärer, gegen Maus-Immunglobulin gerichteter Antikörper, weicher mit Peroxidase gekoppelt war (Dako HRP P 0260; 1:500 verd.) zugegeben und in der feuchten Kammer für 45 Min. bei 37°C inkubiert. Nach erneuten Waschschritten wurde die Anwesenheit von Peroxidase-Aktivität durch die Zugabe von AEC und $H_2O$ nachgewiesen. Anschließend wurde im Umkehrmikroskop die Zahl der Kulturen (Löcher), in welchen HCMV-Antigen nachweisbar war, bei der jeweiligen Verdünnung bestimmt und nach dem unten angegebenen Auswertungsschema die $TCID_{50}$ für die jeweiligen Zellinien und die verschiedenen Zeitpunkte bestimmt.

**TCID 50-Auswertung:**

| Klassengrenze | -9 | -8 | -7 | -6 | -5 | -4 | -3 | -2 | -1 |
|---|---|---|---|---|---|---|---|---|---|
| inf. Kulturen | | | | | | | | | |
| Klassenmitte | | -8.5 | -7.5 | -6.5 | -5.5 | -4.5 | -3.5 | -2.5 | -1.5 |
| Zunahme | | | | | | | | | |
| Produkte: Klassenmitte x Zunahme der Zahl an inf. Kulturen | | | | | | | | | |

**log TCID 50/ 100µl = Summe Produkte / Zahl der Einzelkulturen pro Verdünnung (z.B. 4)**

**Titer TCID 50 /ml = log TCID 50 / 100µl + 1**

Die Ergebnisse der Experimente sind in Abb. 8 dargestellt. Nach Infektion der UL84-negativen Zellinie DC1 kam es beginnend nach 7 Tagen zur Freisetzung von infektiösem Virus in den Kulturüberstand. Nach 12 Tagen nach Infektion ergab sich eine $\mathbf{TCID_{50}}$ von 10.000. Dagegen zeigten die beiden Zellinien IXB6 und IXC5 (UL84-Expression) eine $\mathbf{TCID_{50}}$, weiche um zwei Größenordnungen niedriger lag. Darüber hinaus fand sich in beiden Kulturen kein Anstieg in der Virusproduktion nach 10 und 12 Tagen.

Diese Ergebnisse zeigen, daß die Expression von UL84 in Zellen die Produktion von infektiösem Virus nahezu vollständig inhibiert. Darüber hinaus zeigte sich im Gegensatz zur UL84-negativen Zellinie kein signifikanter Anstieg der Virusproduktion nach 10 und 12 Tagen. Dies deutet darauf hin, daß aufgrund von Variationen in der Expression von UL84 einzelne Zellen in den Linien IXB1 und IXC5 produktiv mit HCMV infiziert sind und geringe Mengen an Virus synthetisieren, daß sich das freigesetzte Virus hier jedoch aufgrund des Replikationsblocks in der überwiegenden Mehrzahl der Zellen der Population nicht vermehren kann.

Insgesamt zeigen damit diese Experimente, daß pUL84 effizient die Replikation des HCMV inhibieren kann.

**Beispiel 6**

**Einführung des UL84-Gens mit Hilfe replikationsdefekter Adenovirus-Vektoren**

Das für das Protein UL84 kodierende Gen kann in die gewünschten Zielzellen mit Hilfe von replikationsdefekten Adenovirus-Vektoren eingebracht werden. Derartige replikationsdefekte Adenovirus-Vektoren wurden z.B. zur Einführung des Retinoblastom-Genprodukts in glatte Gefäßmuskelzellen in vivo eingesetzt, um damit die Entstehung von Restenosen nach PTCA zu verhindern (Chang et al. (1995) Cytostatic gene therapy for vascular proliferative disorders with a constitutively active form of the retinoblastoma gene product, Science 267:518-522). Da HCMV als ätiologisches Agens dieses Prozesses angesehen wird, kann das für UL84 kodierende Gen mit Hilfe der oben erwähnten Adenovirus-Vektoren in die Zellen eingeführt werden. Damit steht eine therapeutische Interventionsmöglichkeit der Entstehung von Restenosen zur Verfügung.

Das replikationsdefekte Adenovirus-Vektorensystem wurde ebenfalls zur Einbringung eines alpha-1-Antitrypsingens in Lungenepithelzellen in vivo und in vitro verwendet (Rosenfeld et al. (1991) Adenovirus-mediated transfer of a recombinant alpha 1-antitrypsin gene to the lung epithelium in vivo, Science 252:431-434). Bei durch HCMV bedingter Pneumonie sind

insbesondere die Epithelzellen infiziert. Die Einführung des Gens für UL84 mit Hilfe der oben beschriebenen Vektoren führt daher zu dem gewünschten therapeutischen Effekt.

**Beispiel 7**

**Einführung des UL84-Gens mit Hilfe Adeno-assoziierter Virusvektoren**

Adeno-assoziierte Virusvektoren wurden von McLaughlin et al. (1988) Adeno-associated virus general transduction vectors: analysis of proviral structures, J. Virol. **62**:1963-1973, beschrieben. Diese Vektoren haben den Vorteil des stabilen Transfers von genetischer Information in Zielzellen, da sie in das zelluläre Genom integrieren.

Solche Vektoren wurden z.B. zum Transfer eines menschlichen Gamma-Globulingens in hämatopoetische Stammzellen verwendet (Miller et al. (1994) Recombinant adeno-associated virus (rAAV)-mediated expression of a human gamma-globin gene in human progenitor-derived erythroid cells, Proc. Natl. Acad. Sci. U.S.A. **91**:10183-10187). Die CMV-induzierten Erkrankungen stellen insbesondere nach Knochenmarkstransplantationen ein erhebliches Problem dar. Die Blutzellen und deren Vorläuferzellen sind aber ein Zielorgan der CMV-Replikation und maßgeblich an der Dissemination des Virus beteiligt. Die Einbringung von UL84 in Knochenmarks-Stammzellen mit Hilfe der oben erwähnten Adeno-assoziierten Virusvektoren stellt eine weitere Ausführungsform der vorliegenden Erfindung dar.

Adeno-assoziierte Virusvektoren (AAV-Vektoren) können auch zur Einbringung von Genen in post-mitotische Zellen, wie z.B. neuronales Gewebe verwendet werden (Kaplitt et al. (1994) Long-term gene expression and phenotypic correction using adeno-associated virus vectors in the mammalian brain, Nat. Genet. **8**:148-154). CMV infiziert vor allem bei AIDS und nach pränataler Infektion unterschiedliche Bereiche des zentralen Nervensystems. Insbesondere eine intraokuläre Einbringung von UL84 mit Adeno-assoziierten Vektoren bei Retinitis erscheint daher erfolgversprechend, da hier die derzeitige medikamentöse Therapie keinen langdauernden Erfolg verspricht.

**Beispiel 8**

**Einführung von UL84 mit Hilfe retroviraler Vektorsysteme**

Retrovirale Vektorsysteme bieten, ähnlich wie Adeno-assoziierte Virusvektoren, den Vorteil der Integration und sind für ein breites Zellspektrum (außer postmitotischen Zellen) anwendbar (Salmons et al. (1993) Targeting of retroviral vectors for gene therapy, Hum. Gene Ther. **4**: 129-141). Die retroviralen Vektorsysteme können für den Transfer von genetischer Information in hämatopoetische Stammzellen oder Zellen des Gastrointestinaltrakts verwendet werden (Bagnis et al. (1994) Retroviral transfer of the nlsLacZ gene into human CD34+ cell populations and into TF-1 cells: future prospects in gene therapy, Hum. Gene Ther. 5:1325-1333; Yoshida, et al. (1995) Retrovirally transmitted gene therapy for gastric carcinoma using herpes simplex virus thymidine kinase gene, Cancer **75**:1467-1471). Die dort beschriebenen retroviralen Vektorsysteme können auch zum Transfer von UL84 eingesetzt werden.

**Beispiel 9**

**Einführung von UL84 mit Hilfe von Liposomen-Transfersystemen**

Liposomen-Transfer wurde insbesondere zur Einführung von Genen in Lungenepithelzellen und Gefäße verwendet (Alton et al. (1993) Non-invasive liposome-mediated gene delivery can correct the ion transport defect in cystic fibrosis mutant mice, Nat. Genet. **5**:135-142; Canonico et al. (1994) Aerosol and intravenous transfection of human alpha 1-antitrypsin gene to lungs of rabbits, Am. J. Respir. Cell Mol. Biol. **10**:24-29; Canonico et al. (1994) No lung toxicity after repeated aerosol or intravenous delivery of plasmid-cationic complexes, J. Appl. Physiol. **77**:415-419; von der Leyen et al. (1995) Gene therapy inhibiting neointimal vascular lesion: in vivo transfer of endothelial cell nitric oxide synthase gene, Proc. Natl. Acad. Sci. U.S.A. **92**:1137-1141). Besonders vorteilhaft dürfte hier die Applikation per Inhalation bei Erkrankungen der Lunge sein.

Mit Hilfe der in den erwähnten Literaturstellen offenbarten Technik kann die CMVinduzierte Pneumonie therapiert werden. Diese Technik kann aber auch zur Einführung von UL84 bei sämtlichen lokalisierten Prozessen verwendet werden, wie beispielsweise bei Hautulzerationen, die durch HCMV hervorgerufen wurden, oder gastrointestinalen Ulzera, die durch HCMV verursacht wurden.

In den angeführten Vektorsystemen ist die Einbringung Zelltyp-spezifischer Promotoren möglich, so daß eine gezielte Expression von UL84 in bestimmten Zellen erreicht werden kann.

Im Rahmen der vorliegenden Erfindung können auch weitere Transfersysteme bzw. Modifikationen der oben näher beschriebenen Transfersysteme zum Einsatz gelangen. Beispielsweise können Liposomen kombiniert mit Sendai-Virus verwendet werden.

**SEQUENZPROTOKOLI**

(1) ALLGEMEINE ANGABEN:

(i) ANMELDER:

(A) NAME: Behringwerke Aktiengesellschaft
(B) STRASSE: Emil-von-Behring-Str. 76
(C) ORT: Marburg
(E) LAND: Deutschland
(F) POSTLEITZAHL: 35041
(G) TELEFON: 06421-39-2205
(H) TELEFAX: 06421-39-4558

(ii) BEZEICHNUNG DER ERFINDUNG: Arzneimittel enthaltend wenigstens einen des UL84-Proteins des Cytomegalovirus, Verwendung von Polypeptiden entsprechend der Aminosaeuresequenz des UL84-Proteins und Verfahren zur Einfuehrung...

(iii) ANZAHL DER SEQUENZEN: 2

(iv) COMPUTER-LESBARE FASSUNG:

(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(vi) DATEN DER URANMELDUNG:

(A) ANMELDENUMMER: DE 19527129.7
(B) ANMELDETAG: 25-JUL-1995

(2) ANGABEN ZU SEQ ID NO: 1:

(i) SEQUENZKENNZEICHEN:

(A) LÄNGE: 39 Basenpaare
(B) ART: Nucleotid
(C) STRANGFORM: Einzelstrang
(D) TOPOLOGIE: linear

(ii) ART DES MOLEKÜLS: Genom-DNA

(iii) HYPOTHETISCH: JA

(iv) ANTISENSE: NEIN

(xi) SEQUENZBESCHREIBUNG: SEQ ID NO: 1:

TAAGAATTCA TGCCACGCGT CGACCCCAAC CTTCGGAAT 39

(2) ANGABEN ZU SEQ ID NO: 2:

(i) SEQUENZKENNZEICHEN:

(A) LÄNGE: 54 Basenpaare
(B) ART: Nucleotid
(C) STRANGFORM: Einzelstrang
(D) TOPOLOGIE: linear

(ii) ART DES MOLEKÜLS: Genom-DNA

(iii) HYPOTHETISCH: JA

(iv) ANTISENSE: NEIN

(xi) SEQUENZDESCHREIBUNG: SEQ ID NO: 2:

TAATCTAGAT CCCTAGGTAC CTTCGAGATC GCCGCAGACC ATGGCTAAAG TGAC 54

## Patentansprüche

**1.** Arzneimittel, **dadurch gekennzeichnet, daß** es

a) ein Polypeptid mit einer Sequenz, die wenigstens die 180 N-terminalen Aminosäuren des Proteins UL84 des Cytomegalovirus umfaßt, oder
b) eine zu der Sequenz des Polypeptids in Teil a) wenigstens zu 80% homologe Sequenz umfaßt.

**2.** Arzneimittel nach Anspruch 1, **dadurch gekennzeichnet, daß** das Polypeptid aus den 180 N-terminalen Aminosäuren von UL84 oder einer Sequenz mit wenigstens 80% Homologie dazu besteht.

**3.** Arzneimittel nach Anspruch 2, **dadurch gekennzeichnet, daß** das Polypeptid aus den 180 N-terminalen Aminosäuren von UL84 besteht.

**4.** Arzneimittel nach Anspruch 1, **dadurch gekennzeichnet, daß** das Polypeptid aus der gesamten UL84-Kodierungssequenz oder einer Sequenz mit wenigstens 80% Homologie dazu besteht.

5. Arzneimittel nach Anspruch 4, **dadurch gekennzeichnet, daß** das Polypeptid aus der gesamten UL84-Aminosäuresequenz besteht.

6. Arzneimittel nach einem der Ansprüche 1-4, **dadurch gekennzeichnet, daß** die UL84-Aminosäuresequenz der Darstellung in Abbildung 1 entspricht.

7. Polypeptid gemäß der Definition der Ansprüche 1-6 zur Verwendung als Medikament.

8. Verwendung eines Polypeptids gemäß der Definition der Ansprüche 1-6 bei der Herstellung eines Medikaments zur Prophylaxe oder Behandlung einer Infektion von humanem Cytomegalovirus.

## Claims

1. Pharmaceutical, **characterized in that** it comprises

    a) a polypeptide having a sequence which comprises at least the 180 N-terminal amino acids of the cytomegalovirus protein UL84, or
    b) a sequence which is at least 80% homologous to the sequence of the polypeptide in part a).

2. Pharmaceutical according to Claim 1, **characterized in that** the polypeptide is composed of the 180 N-terminal amino acids of UL84 or is composed of a sequence having at least 80% homology thereto.

3. Pharmaceutical according to Claim 2, **characterized in that** the polypeptide is composed of the 180 N-terminal amino acids of UL84.

4. Pharmaceutical according to Claim 1, **characterized in that** the polypeptide is composed of the entire UL84-encoding sequence or is composed of a sequence having at least 80% homology thereto.

5. Pharmaceutical according to Claim 4, **characterized in that** the polypeptide is composed of the entire UL84 amino acid sequence.

6. Pharmaceutical according to one of Claims 1-4, **characterized in that** the UL84 amino acid sequence corresponds to the depiction in Figure 1.

7. Polypeptide according to the definition of Claims 1-6 for use as a medicament.

8. Use of a polypeptide according to the definition of Claims 1-6 for producing a medicament for the prophylaxis or treatment of an infection due to human cytomegalovirus.

## Revendications

1. Médicament **caractérisé en ce que**

    a) il contient un polypeptide présentant une séquence qui contient au moins les 180 acides aminés N-terminaux de la protéine UL84 du cytomégalovirus, ou
    b) une séquence homologue à au moins 80 % à la séquence du polypeptide de a).

2. Médicament selon la revendication 1, **caractérisé en ce que** le polypeptide est constitué des 180 acides aminés N-terminaux d'UL84 ou d'une séquence ayant au moins 80 % d'homologie avec cette séquence.

3. Médicament selon la revendication 2, **caractérisé en ce que** le polypeptide est constitué des 180 acides aminés N-terminaux d'UL84.

4. Médicament selon la revendication 1, **caractérisé en ce que** le polypeptide est constitué de l'ensemble de la séquence codante d'UL84 ou d'une séquence ayant au moins 80 % d'homologie avec cette séquence.

5. Médicament selon la revendication 4, **caractérisé en ce que** le polypeptide est constitué de l'ensemble de la séquence d'acides aminés d'UL84.

6. Médicament selon l'une des revendications 1-4, **caractérisé en ce que** la séquence d'acides aminés d'UL84 correspond à la représentation de la figure 1.

7. Polypeptide selon la définition des revendications 1 à 6 destiné à être utilisé comme médicament.

8. Utilisation d'un polypeptide selon la définition des revendications 1 à 6 dans la fabrication d'un médicament pour la prophylaxie ou le traitement d'une infection par le cytomégalovirus humain.

```
ATG CCA CGC GTC GAC CCC AAC CTT CGG AAT CGG GCC CGC CGG CCA CGA      48
Met Pro Arg Val Asp Pro Asn Leu Arg Asn Arg Ala Arg Arg Pro Arg
 1               5                  10                  15

GCC AGA CGA GGC GGC GGC GGT GGC GTT GGC AGC AAT AGC AGC CGA CAC      96
Ala Arg Arg Gly Gly Gly Gly Gly Val Gly Ser Asn Ser Ser Arg His
            20                  25                  30

AGC GGA AAA TGC CGC CGC CAA CGC CGA GCT CTG TCG GCG CCG CCG CTC     144
Ser Gly Lys Cys Arg Arg Gln Arg Arg Ala Leu Ser Ala Pro Pro Leu
        35                  40                  45

ACT TTC CTC GCC ACC ACT ACC ACC ACG ACC ATG ATG GGC GTC GCC AGT     192
Thr Phe Leu Ala Thr Thr Thr Thr Thr Thr Met Met Gly Val Ala Ser
    50                  55                  60

ACC GAC GAC GAC AGT CTC CTC CTG AAA ACG CCG GAC GAG CTG GAC AAG     240
Thr Asp Asp Asp Ser Leu Leu Leu Lys Thr Pro Asp Glu Leu Asp Lys
65                  70                  75                  80

TAC AGC GGC TCG CCG CAG ACC ATC CTC ACA CTG ACG GAT AAA CAC GAC     288
Tyr Ser Gly Ser Pro Gln Thr Ile Leu Thr Leu Thr Asp Lys His Asp
                85                  90                  95

ATC CGT CAG CCT CGG GTG CAC CGC GGC ACC TAC CAT CTG ATC CAG TTG     336
Ile Arg Gln Pro Arg Val His Arg Gly Thr Tyr His Leu Ile Gln Leu
           100                 105                 110

CAC CTC GAC CTC CGA CCC GAA GAA TTG CGG GAT CCC TTC CAG ATT CTG     384
His Leu Asp Leu Arg Pro Glu Glu Leu Arg Asp Pro Phe Gln Ile Leu
       115                 120                 125
```

Abb. 1a

```
CTC TCT ACG CCG CTG CAA TTG GGG GAA GCG AAC GAC GAG TCT CAA ACC     432
Leu Ser Thr Pro Leu Gln Leu Gly Glu Ala Asn Asp Glu Ser Gln Thr
    130                 135                 140

GCC CCC GCG ACG TTG CAA GAA GAA GAA ACG GCG GCT TCC CAC GAG CCC     480
Ala Pro Ala Thr Leu Gln Glu Glu Glu Thr Ala Ala Ser His Glu Pro
145                 150                 155                 160

GAG AAA AAA AAG GAA AAA CAA GAG AAG AAA GAA GAG GAC GAG GAT GAC     528
Glu Lys Lys Lys Glu Lys Gln Glu Lys Lys Glu Glu Asp Glu Asp Asp
                165                 170                 175

CGC AAC GAC GAT CGT GAA CGC GGC ATT CTA TGC GTG GTC TCT AAC GAG     576
Arg Asn Asp Asp Arg Glu Arg Gly Ile Leu Cys Val Val Ser Asn Glu
            180                 185                 190

GAT TCT GAC GTG CGC CCG GCC TTC TCT CTC TTT CCC GCA CGC CCA GGC     624
Asp Ser Asp Val Arg Pro Ala Phe Ser Leu Phe Pro Ala Arg Pro Gly
        195                 200                 205

TGC CAT ATC CTG CGC TCG GTA ATT GAC CAA CAA CTG ACG CGC ATG GCC     672
Cys His Ile Leu Arg Ser Val Ile Asp Gln Gln Leu Thr Arg Met Ala
    210                 215                 220

ATC GTG CGC CTA TCA CTC AAT CTC TTC GCG CTC CGT ATC ATC ACG CCG     720
Ile Val Arg Leu Ser Leu Asn Leu Phe Ala Leu Arg Ile Ile Thr Pro
225                 230                 235                 240

CTG TTG AAA CGG CTA CCG CTA CGA CGT AAA GCC GCG CAT CAC ACG GCG     768
Leu Leu Lys Arg Leu Pro Leu Arg Arg Lys Ala Ala His His Thr Ala
                245                 250                 255
```

Abb.1b

```
TTA CAC GAC TGT CTG GCG CTG CAT CTG CCA GAA CTC ACG TTC GAG CCG      816
Leu His Asp Cys Leu Ala Leu His Leu Pro Glu Leu Thr Phe Glu Pro
            260                 265                 270

ACG CTG GAT ATA AAC AAC GTA ACG GAG AAC GCG GCT TCC GTC GCT GAT      864
Thr Leu Asp Ile Asn Asn Val Thr Glu Asn Ala Ala Ser Val Ala Asp
        275                 280                 285

ACC GCG GAA TCA ACG GAC GCG GAT CTG ACG CCC ACG CTG ACG GTG CGC      912
Thr Ala Glu Ser Thr Asp Ala Asp Leu Thr Pro Thr Leu Thr Val Arg
    290                 295                 300

GTA CGA CAC GCG CTG TGC TGG CAT CGA GTG GAA GGC GGC ATC TCG GGG      960
Val Arg His Ala Leu Cys Trp His Arg Val Glu Gly Gly Ile Ser Gly
305                 310                 315                 320

CCG CGT GGA CTC ACC AGC CGT ATC TCG GCG CGC CTC TCG GAA ACC ACG     1008
Pro Arg Gly Leu Thr Ser Arg Ile Ser Ala Arg Leu Ser Glu Thr Thr
                325                 330                 335

GCC AAG ACA TTG GGA CCC TCC GTC TTT GGA CGA TTG GAG CTA GAC CCG     1056
Ala Lys Thr Leu Gly Pro Ser Val Phe Gly Arg Leu Glu Leu Asp Pro
            340                 345                 350

AAC GAA TCA CCG CCG GAC CTG ACG CTG TCG TCA CTC ACG CTA TAC CAA     1104
Asn Glu Ser Pro Pro Asp Leu Thr Leu Ser Ser Leu Thr Leu Tyr Gln
        355                 360                 365

GAC GGC ATA TTA CGT TTC AAC GTG ACC TGC GAC CGC ACC GAG GCG CCA     1152
Asp Gly Ile Leu Arg Phe Asn Val Thr Cys Asp Arg Thr Glu Ala Pro
    370                 375                 380
```

Abb.1c

```
GCC GAC CCA GTG GCG TTT CGC CTG CGG CTG CGA CGC GAA ACG GTG CGA     1200
Ala Asp Pro Val Ala Phe Arg Leu Arg Leu Arg Arg Glu Thr Val Arg
385                 390                 395                 400

CGA CCC TTC TTT TCG GAC GCG CCA CTG CCT TAC TTT GTA CCG CCA CGC     1248
Arg Pro Phe Phe Ser Asp Ala Pro Leu Pro Tyr Phe Val Pro Pro Arg

                405                 410                 415

TCC GGC GCG GCG GAC GAG GGA CTG GAG GTG CGC GTC CCT TAC GAA TTG     1296
Ser Gly Ala Ala Asp Glu Gly Leu Glu Val Arg Val Pro Tyr Glu Leu
            420                 425                 430

ACG CTG AAG AAC TCG CAC ACG TTA CGT ATC TAC CGC CGC TTT TAC GGG     1344
Thr Leu Lys Asn Ser His Thr Leu Arg Ile Tyr Arg Arg Phe Tyr Gly
        435                 440                 445

CCT TAT CTG GGT GTT TTT GTA CCA CAC AAC CGT CAG GGA CTC AAA ATG     1392
Pro Tyr Leu Gly Val Phe Val Pro His Asn Arg Gln Gly Leu Lys Met
    450                 455                 460

CCC GTT ACG GTC TGG CTA CCG CGC TCC TGG TTG GAA TTA ACC GTA CTG     1440
Pro Val Thr Val Trp Leu Pro Arg Ser Trp Leu Glu Leu Thr Val Leu
465                 470                 475                 480

GTG AGC GAC GAG AAC GGC GCC ACG TTC CCA CGG GAC GCG CTC CTG GGG     1488
Val Ser Asp Glu Asn Gly Ala Thr Phe Pro Arg Asp Ala Leu Leu Gly
                485                 490                 495
```

Abb.1d

```
CGC CTC TAT TTT ATC TCG TCA AAG CAT ACG CTG AAT CGG GGT TGC CTG    1536
Arg Leu Tyr Phe Ile Ser Ser Lys His Thr Leu Asn Arg Gly Cys Leu
            500                 505                 510

TCA GCA ATG ACG CAC CAA GTC AAA TCC ACG CTA CAC TCG CGG TCC ACA    1584
Ser Ala Met Thr His Gln Val Lys Ser Thr Leu His Ser Arg Ser Thr
        515                 520                 525

TCC CAT TCG CCG TCG CAA CAG CAG CTC TCG GTG CTG GGC GCT TCC ATC    1632
Ser His Ser Pro Ser Gln Gln Gln Leu Ser Val Leu Gly Ala Ser Ile
    530                 535                 540

GCG CTG GAG GAC CTG CTG CCC ATG CGA CTG GCG TCC CCG GAG ACG GAA    1680
Ala Leu Glu Asp Leu Leu Pro Met Arg Leu Ala Ser Pro Glu Thr Glu
545                 550                 555                 560

CCG CAA GAC TGT AAG CTT ACG GAA AAT ACG ACA GAG AAG ACG AGT CCT    1728
Pro Gln Asp Cys Lys Leu Thr Glu Asn Thr Thr Glu Lys Thr Ser Pro
                565                 570                 575

GTC ACT TTA GCC ATG GTC TGC GGC GAT CTC TAA                        1761
Val Thr Leu Ala Met Val Cys Gly Asp Leu  *
            580                 585
```

Abb. 1e

Abb. 2: Schematische Darstellung des eukaryonten Expressionsplasmids pcDNA-UL84

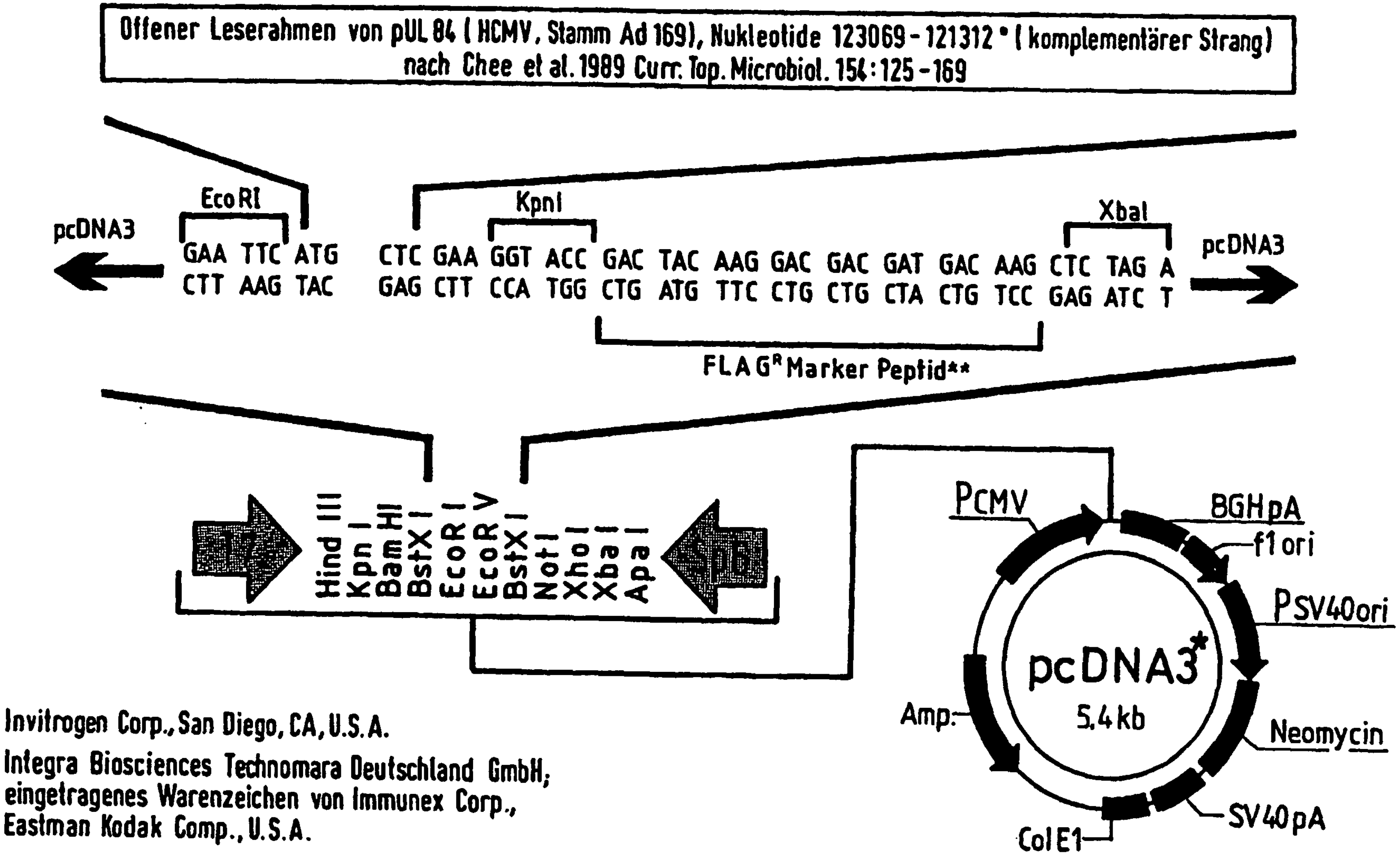


* Invitrogen Corp., San Diego, CA, U.S.A.

** Integra Biosciences Technomara Deutschland GmbH; eingetragenes Warenzeichen von Immunex Corp., Eastman Kodak Comp., U.S.A.

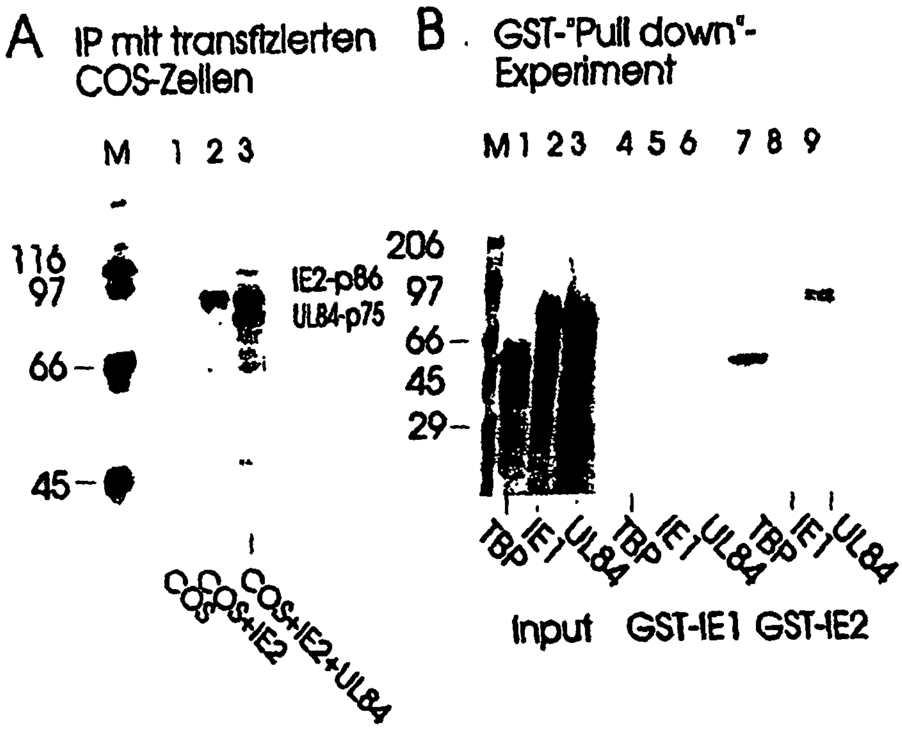
A IP mit transfizierten COS-Zellen
M 1 2 3
116
97
66
45
IE2-p86
UL84-p75
COS
COS+IE2
COS+IE2+UL84
B GST-"Pull down"-Experiment
M 1 2 3 4 5 6 7 8 9
206
97
66
45
29
TBP
IE1
UL84
TBP
IE1
UL84
TBP
IE1
UL84
Input
GST-IE1
GST-IE2

FIG. 3

**Abb. 4: Einfluß von pUL84 auf die IE2-vermittelte Transaktivierung homologer und heterologer viraler Promotoren**

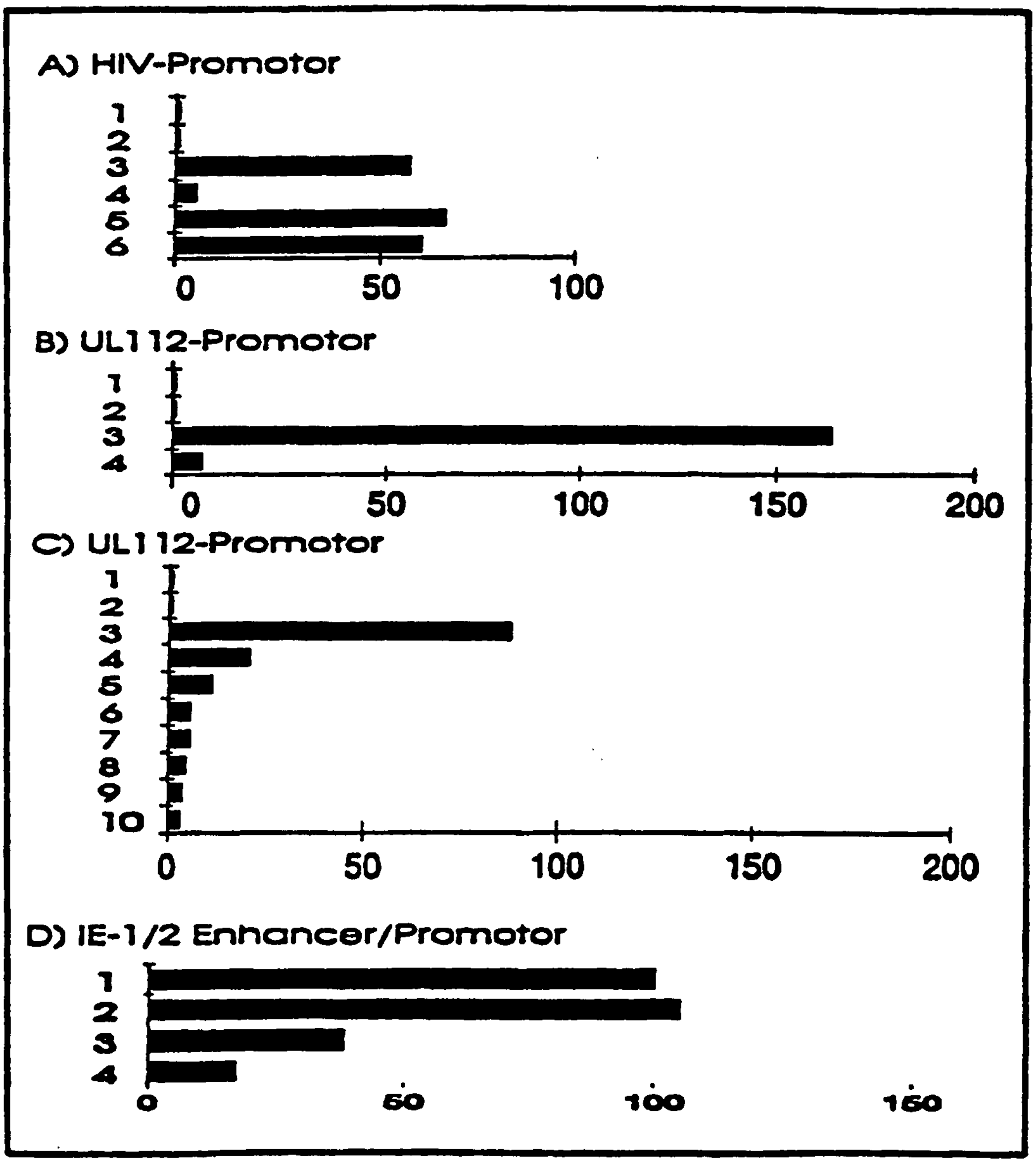


Abb. 4

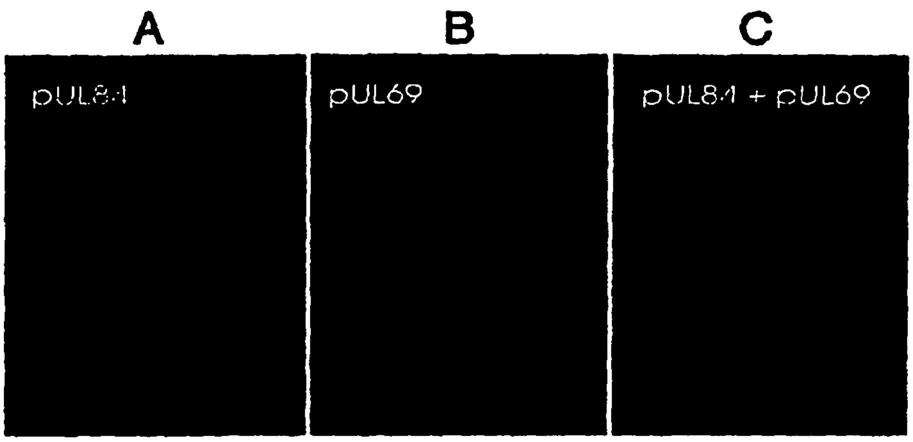


FIG. 5

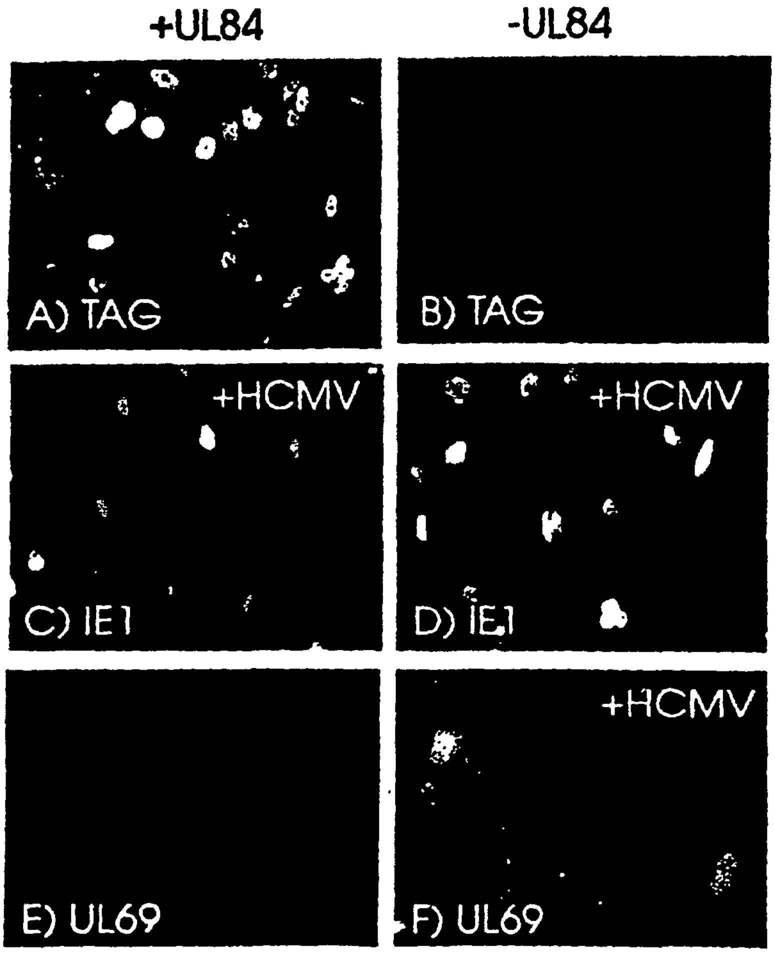


FIG. 6

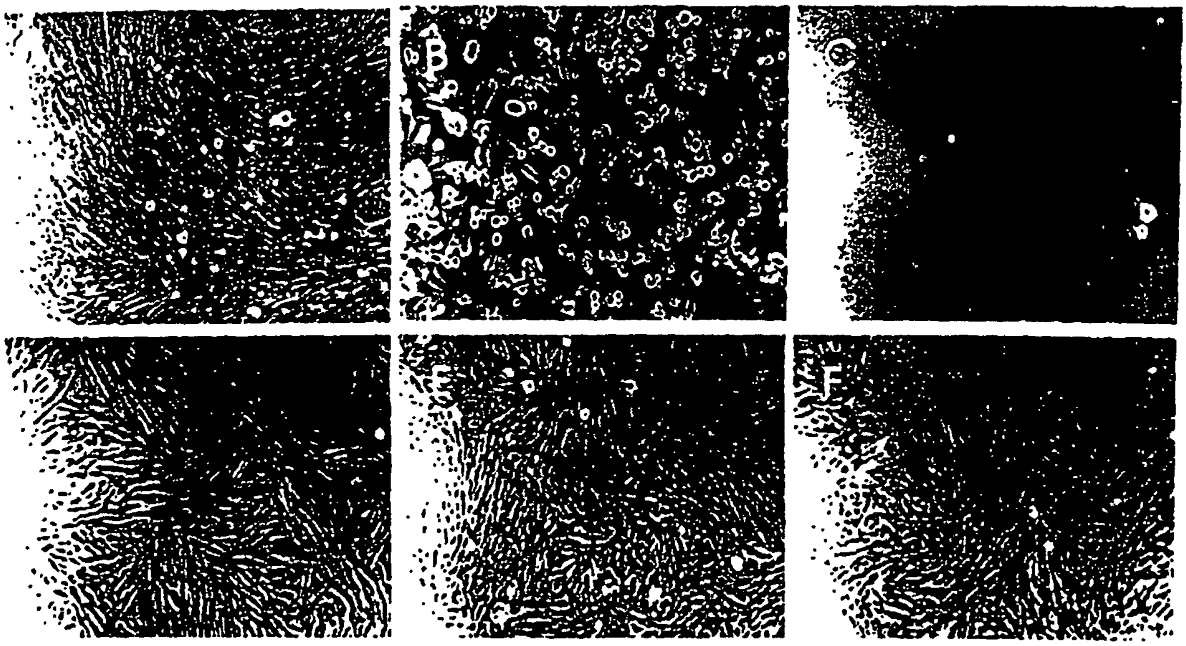


FIG. 7

**Abb. 8: Hemmung der Virusvermehrung nach Infektion von UL84-exprimierenden Zellinien mit HCMV**

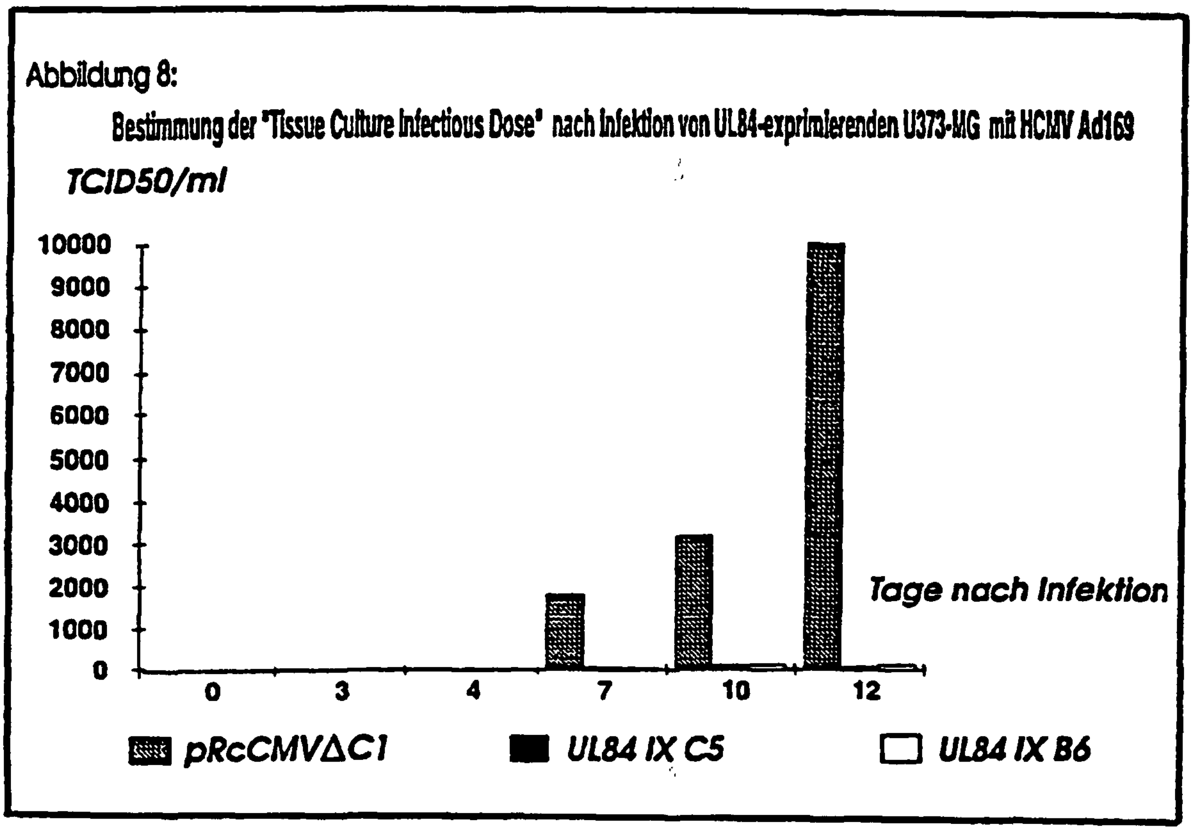


Abb.8